(19) Europäisches Patentamt
European Patent Office
Office européen des brevets

(11) **EP 2 876 122 A1**

(12) **EUROPEAN PATENT APPLICATION**

(43) Date of publication:
**27.05.2015 Bulletin 2015/22**

(21) Application number: **14192129.6**

(22) Date of filing: **06.11.2014**

(51) Int Cl.:
*C08G 59/22* (2006.01)     *C08J 3/00* (2006.01)
*C08G 59/24* (2006.01)     *C09D 163/00* (2006.01)
*C08L 63/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.11.2013 US 201361906573 P**

(71) Applicant: **Dow Global Technologies LLC**
**Midland, MI 48674 (US)**

(72) Inventors:
• **Drumright, Ray E.**
  **Midland, Michigan 48640 (US)**
• **Hefner, Jr., Robert E.**
  **Rosharon, Texas 77583 (US)**

(74) Representative: **Houghton, Mark Phillip**
**Patent Outsourcing Limited**
**1 King Street**
**Bakewell, Derbyshire DE45 1DZ (GB)**

(54) **Low-viscosity epoxy resins and low voc curable formulations therefrom**

(57)     The present invention provides methods of making a low viscosity $C_{15}$ alkenyl group containing diglycidyl ether composition comprising at least partially distilling a mixture of one or more $C_{15}$ alkenyl group containing monoglycidyl ether and one or more $C_{15}$ alkenyl group containing diglycidyl ether in the presence of a free radical inhibitor to remove at least some of the $C_{15}$ alkenyl group containing monoglycidyl ether. The methods enable high solids, low viscosity epoxy resins from a renewable source. The present invention also provides compositions of $C_{15}$ alkenyl group containing diglycidyl ether adducts of a polyamine.

**EP 2 876 122 A1**

**Description**

The present invention relates to methods for making low viscosity epoxy resins from a renewable source comprising $C_{15}$ alkenyl group containing diglycidyl ethers and methods for making low volatile organic compound (VOC) liquid coating compositions from the low viscosity $C_{15}$ alkenyl group containing diglycidyl ethers. Further, the present invention relates to methods of making the polyamine adducts of the C15 alkenyl group containing diglycidyl ethers; and it relates to the polyamine adducts and the coating compositions made therefrom. More particularly, it relates to methods comprising separating a mixture of one or more $C_{15}$ alkenyl group containing monoglycidyl ether and one or more $C_{15}$ alkenyl group containing diglycidyl ether by distillation in the presence of a free radical inhibitor to fractionate the diglycidyl ether from the monoglycidyl ether to provide epoxy resin compositions having a surprisingly low viscosity.

Previously, coating applicators have dealt with the coating composition applicability problems of epoxy coatings, i.e. the sprayability or paintability, by adding one or more organic solvents or one or more reactive diluents, or using heated application equipment. The solvents, e.g. xylene, are generally polluting, and are considered VOCs as they evaporate in use. The diluents, usually organic compounds with active hydrogens (e.g., alcohol such as benzyl alcohol) or epoxy functional compounds such as cresol glycidyl ether or butanediol diglycidyl ether, can be incompletely reacted into the epoxy coating during cure and thereby become VOCs and/or lead to deterioration in properties in the final coating due to disruption of crosslinking and/or plasticization of the coating film. Heated plural component application equipment is difficult to use in the field and is expensive to buy and maintain.

Japanese patent publication no. 10-218973A, to Asahi Chemical Ind. Co. Ltd., discloses low VOC epoxy resin compositions that comprise epoxy resins made from cardols mixed with other conventional epoxy resins, wherein the $C_{15}$ alkyl or alkenyl side chain of the cardols used to make the epoxy resins have an average of 0.5 or more double bonds, and wherein the cardol compositions used to make the epoxy resins comprises 90 wt. % or more of cardol. Without the stated cardol purity, the disclosure indicates that coatings or products from the composition suffer from discoloration or curing and/or flexibility is adversely impacted. In the epoxy resin made from the cardol, the side chain comprises about 1 wt. % of a $C_{15}$ alkyl or saturated substituent. Also disclosed are two component coating compositions comprising a curative or polyamine. The disclosure emphasizes the importance of using highly purified cardols which requires additional refining of a cardol mixture containing cardanol to remove the cardanol and, further, to remove saturated $C_{15}$ alkyl side chains from the cardol mixture prior to forming an epoxy resin therefrom. In addition, the Working Examples disclose reacting the cardols for over 7 hours at 100°C. The resulting resin compositions cure at room temperature in 24 hours.

The present inventors have sought to solve the problem of efficiently providing low viscosity epoxy resins from renewable $C_{15}$ alkenyl aromatic diglycidyl ether mixtures having up to 25 wt. % of cardanol epoxy and yet a low viscosity of from 50 to 1800 cPs at 25°C and to solve the problem of providing a more rapid cure low VOC epoxy composition therefrom.

STATEMENT OF THE INVENTION

1. In accordance with the present invention, methods of forming low viscosity epoxy resin compositions comprise distilling a mixture of one or more $C_{15}$ alkenyl group containing monoglycidyl ether and one or more $C_{15}$ alkenyl group containing diglycidyl ether, preferably in the presence of a free radical inhibitor, to at least partially fractionate the diglycidyl ether from the monoglycidyl ether. The resulting epoxy resin compositions may have a viscosity of from 50 to 2000 cPs at 25 °C, or, preferably, from 100 to 1800 cPs or, more preferably, from 100 to 1500 cPs.

2. In accordance with the methods of 1, above, the free radical inhibitor is chosen from a phenol having one or two bulky groups *ortho* to the OH group such as two bulky tertiary alkyl groups or an aryl benzofuranone. Preferably, the free radical addition inhibitor is 2,6-di-tertiary-butyl-4-methylphenol (BHT).

3. In accordance with the methods of any of 1 or 2, above, the mixture of one or more $C_{15}$ alkenyl group containing monoglycidyl ether and one or more $C_{15}$ alkenyl group containing diglycidyl ether has up to 30 wt. %, or, preferably, up to 25 wt.%, or, more preferably, from 0.01 to 20 wt. % based on the total solids of the mixture of epoxy resin, of a $C_{15}$ alkenyl group containing monoglycidyl ether.

4. In accordance with the methods of any of 1, 2 or 3, above, the methods further comprising forming the mixture of one or more $C_{15}$ alkenyl group containing monoglycidyl ether and one or more $C_{15}$ alkenyl group containing diglycidyl ether by epoxidizing a mixture of one or more cardol and one or more cardanol with an epihalohydrin, preferably, epichlorohydrin prior to the distilling.

5. In accordance with the methods of any of 1, 2, 3 or 4, above, the resulting epoxy resin composition is substantially free of oligomers resulting from side chain coupling reactions and comprises 15 wt.% or less of oligomers resulting from side chain coupling reactions of alkenyl groups, or, preferably, 10 wt.% or less, or, more preferably, 5 wt.% or less, based on the total solids of the given epoxy resin composition.

6. In accordance with the methods of any of 1, 2, 3, 4 or 5, above, the methods further comprising reacting the one or more $C_{15}$ alkenyl group containing diglycidyl ether with a polyamine, preferably, diethylenetriamine, to form a polyamine adduct of the $C_{15}$ alkenyl group containing diglycidyl ether.

7. In another aspect of the present invention, low viscosity and low VOC epoxy resin compositions comprise the adduct of a polyamine and one or more $C_{15}$ alkenyl group containing diglycidyl ether.

8. In accordance with the compositions of 7, above, rapid cure liquid coating compositions comprise the adduct of a polyamine and the one or more $C_{15}$ alkenyl group containing diglycidyl ether and have a solids content of 85 wt. % solids or higher, preferably, 95 wt.% or higher, e.g. 100 wt. % solids, based on the total weight of the composition, wherein the compositions can be easily applied as field coatings to substrates.

9. In accordance with the compositions of 8, above, the resulting coating composition has an initial viscosity of 50 to 3000 cPs at 25°C, preferably, 100 to 2000 cPs.

Unless otherwise indicated, conditions of temperature and pressure are ambient temperature and standard pressure. All ranges recited are inclusive and combinable.

Unless otherwise indicated, any term containing parentheses refers, alternatively, to the whole term as if no parentheses were present and the term without them, and combinations of each alternative. Thus, the term "(poly)alkoxy" refers to alkoxy, polyalkoxy, or mixtures thereof.

All ranges are inclusive and combinable. For example, the term "a range of 50 to 3000 cPs, or 100 or more cPs" would include each of 50 to 100 cPs, 50 to 3000 cPs and 100 to 3000 cPs.

As used herein, the term "AHEW" refers to the amine hydrogen equivalent weight of an amine containing resin, e.g., an adduct, or hardener, as determined by weighing a 0.1 - 0.15 g sample into glass bottle and adding a magnetic stirring bar and glacial acetic acid (40 mL) followed by stirring to give a solution. Adding six drops of crystal violet solution (0.1 % wt in glacial acetic acid) to the stirred solution followed by titration with 0.1 N perchloric acid to an endpoint indicated by a sharp color change from blue to green that is stable for 2 min. The weight percent amine nitrogen (AmN) in the sample is calculated as follows:

(normality of perchloric acid titrant) (mL perchloric acid used to titrate sample) (1.4) (g sample used)

Then AHEW is calculated as follows:

$$AHEW = \frac{1}{\frac{AmN}{(\% Np)(AHEWp)} - \frac{1-(AmN / \% Np)}{EEW}}$$

Wherein, Np is the wt.% nitrogen in polyamine reactant; AHEWp is the amine hydrogen equivalent weight of the polyamine reactant; and EEW is the epoxide equivalent weight of the epoxy resin reactant.

As used herein, the term "alkenyl containing" refers to a molecule that has a chemical substituent which contains an alkenyl group, such as, for example, an alkenyl side chain.

As used herein, the term "EEW" refers to the epoxy equivalent weight of an epoxy resin or adduct thereof as indicated by titration using the method outlined in the Examples, below.

As used herein, the term "viscosity" refers to the end result as determined by measurement of a epoxy resin composition having the indicated wt.% solids on a Stabinger Viscometer (Model SVM 3000, Anton Paar (Ashland, VA)) at 25°C. For epoxy resins themselves, the wt.% solids is 100%; for coating compositions, the wt.% solids can go as low as 85 wt.%.

As used herein, the term "initial viscosity" refers to the viscosity of a coating composition of the epoxy component and the hardener component measured directly after mixing the two components.

As used herein, the term "low VOC" means a composition having less than 20 wt.% of any VOC, preferably, less than 15 wt%, or, more preferably, less than 10 wt.%, or less than 5 wt.%.

As used herein, unless otherwise indicated, the term "solids content" refers to the total weight of epoxy resins, hardeners, catalysts or accelerators, and other non-volatile materials, such as pigments, silicones and non-volatile additives, expressed as a total wt.% of the coating composition. Solids exclude solvents, such as xylene, and nonreactive diluents, such as, for example, plasticizers like butyl adipates.

As used herein, the term "substantially free of oligomers resulting from side chain coupling reactions of alkenyl groups" means that a given epoxy resin composition comprises 15 wt.% or less of oligomers resulting from side chain coupling reactions of such alkenyl groups, or, preferably, 10 wt.% or less, or, more preferably, 5 wt.% or less, based on the total solids of the given epoxy resin composition.

As used herein, the abbreviation "wt. %" stands for weight percent.

The present invention provides epoxy resins from a renewable source as a low viscosity epoxy resin, comprising fractionated cardol diglycidyl ethers. The epoxy resins are substantially free of oligomers resulting from side chain coupling reactions and are the result of distilling the $C_{15}$ alkenyl group containing diglycidyl ether composition or a mixture thereof with one or more $C_{15}$ alkenyl group containing monoglycidyl ether in the presence of a free radical inhibitor. The fractionated, purified cardol diglycidyl ether gives a remarkable viscosity at 25 °C of only approximately 640 cP; this viscosity is lower in mixtures containing more of the $C_{15}$ alkenyl group containing monoglycidyl ether. The epoxy resins are room temperature curable with a polyamine, e.g. diethylenetriamine (DETA). Further, the cured thermoset - has a - high Tg (60 °C for the DETA cured system). The compositions have utility in anticorrosive coatings, chemical resistant coatings, composites, and adhesive applications. In addition, the compositions are bisphenol A free. The low viscosity epoxy resins could also be used as a reactive epoxy functional diluent in conventional epoxy resins and formulations containing said epoxy resins.

In the distillation of the mixture of a $C_{15}$ alkenyl group containing diglycidyl ether composition with one or more $C_{15}$ alkenyl group containing monoglycidyl ether, a free radical inhibitor may be used but then removed at a later stage of the distillation to produce a controlled amount of oligomerization. It is also operable to use a combination of two or more free radical inhibitors which are partially or totally removed during the distillation at different rates.

The present invention also provides a polyamine adduct of $C_{15}$ alkenyl group containing diglycidyl ether that provides an improved rapid, low temperature cure profile, particularly reduction of cure energy. The $C_{15}$ hydrocarbon chain inherent to the cardol (or cardol / cardanol) epoxy resin adducts of the present invention provides hydrophobic character to increase moisture resistance and provide toughened thermoset matrices.

The cardol or cardol/cardanol mixtures that are epoxidized in the present invention are obtained from processing of spent cashew nut shells and are widely available as commercial products. Such compositions are refined from the husks of cashew nut shells as disclosed, for example, in U.S. Patent No. 2,985,622 to Leppert. The cashew nut shell liquid occurs naturally in the husk of the fruit of the cashew tree *(Anacardium occidentale* or the genus *Anacardiaceae)* and consists chiefly of anacardic acid compounds having a 15-carbon ($C_{15}$) straight, unsaturated side chain with from 0 to about 3 double bonds per chain. Also present in the liquid is about 10% or more of cardol, a dihydroxybenzene having the $C_{15}$ side chain. Useful cashew nut shell liquids (CNSL) may be obtained by extracting the oil from ground husks in a hot bath of previously extracted oil. During the hot extraction most of the anacardic acid is decarboxylated and some polymerization occurs, so that the extracted liquid consists of mono- and di-phenolic olefins with a carbon skeleton of 3-pentadecyl phenol and 5-pentadecyl resorcinol. The extracted liquid may be detoxicated by heating the extracted liquid with agents such as fluoroboric acid, sulphuric acid, phosphoric acid, hydrochloric acid, diethyl sulphate, or the like in order to eliminate a toxic effect analogous to that of poison ivy and to purify the oil by precipitation of an undefined sludge. Small amounts of anacardic acid, polymers and husk contaminants may be present so that the product is an oily, dark brown liquid.

In the present invention, cardol or a cardol/cardanol mixture is epoxidized and then distilled to remove some or all of the $C_{15}$ alkenyl group containing monoglycidyl ether and low boiling point impurities in the presence of free radical addition inhibitor. Such distilling prevents oligomerization through the side chain. Suitable free radical inhibitors may include phenols with one or more bulky groups *ortho* to the OH group, such as, for example, 2,6-di-tertiary-butyl-4-methylphenol (BHT), 2,4-dimethyl-6-tert-butylphenol (Ionol), tetrakis-(methylene-(3,5-di- tert- butyl-4-hydrocinnamate)methane or 4,4'-butylidenebis(6-tert-butyl-m-cresol). Oligomers or polymers of hindered phenolic monomers, such as 4-methylene(3,5-di-tert-butyl-4-phenoxy)styrene or 6-tert-butyl-2-(1,1-dimethyl-hept-6-enyl)-4-methylphenol, may be used. The 3-Aryl-benzofuranones, for example Irganox™ HP-136 (Ciba Specialty Chemicals Inc., Basel, CH), may be used.

Suitable amounts of the free radical inhibitor may range from 0.01 to 5 wt.%, based on total epoxy resin solids, preferably from 0.1 to 2 wt.%.

Distillation may be performed by heating of a $C_{15}$ alkenyl group containing monoglycidyl ether with application of a vacuum to distill off the one or more $C_{15}$ alkenyl group containing monoglycidyl ether and lower boiling impurities, or a combination thereof leaving behind an epoxy resin enriched in $C_{15}$ alkenyl group containing diglycidyl ether. In distilling the mixture, the heating temperature may range in temperature of from of 125 to 260 °C, or, preferably, from 150 °C to 250°C.

Suitable distillation methods are well known to those skilled in the art. One suitable distillation method comprises short path vacuum distillation with an unpacked glass column. Other methods include falling film and wiped film distillations. One or more distillation methods may be used in combination.

The epoxidation of a mixture of one or more cardol and one or more cardanol comprises reacting the mixture with an epihalohydrin. Suitable amounts of epihalohydrin range from a 1:1 ratio to a 20:1 ratio of epihalohydrin equivalents to hydroxyl equivalents, preferably from 3:1 to 8:1. One or more basic acting substances, e.g., sodium hydroxide, are employed in the epoxidation. One or more catalysts such as quaternary ammonium or phosphonium salts may optionally be employed. One or more solvents, e.g., isopropanol, may optionally be employed. The epoxidation reaction may optionally be an azeotropic epoxidation wherein an epichlorohydrin and water azeotrope is removed under vacuum during the reaction.

Various CNSL mixtures from may be epoxidized, including those having up to 75 wt. % cardanol, or, 25 wt. % of cardanol, or, up to 15 wt. %, or, preferably, from 0.01 to 10 wt. %, based on total solids of cardol and cardanol. When higher amounts of cardanol are in the CNSL mixtures being epoxidized, the result will be lower functionality of the product epoxy resin but also lower viscosity of the epoxy resin product.

The epoxy resin comprising a $C_{15}$ alkenyl group containing diglycidyl ether of the present invention can be reacted with a polyamine to obtain an adduct suitable for use in rapid curing two component coating compositions. Preferably, the $C_{15}$ alkenyl group containing diglycidyl ether is substantially free of oligomer from side chain coupling reactions.

The adducts are formed by simply mixing and heating the epoxy resin and a stoichiometric excess of one or more polyamine. Excess polyamine may then be removed from the adduct or a part or all may be left in the adduct.

Suitable polyamines, include, for example, ethyleneamines such as diethylenetriamine, triethylenetetramine, bis(2-(piperazin-1-yl)ethylamine, aminoethylpiperazine; other polyalkylenepolyamines, cycloaliphatic diamines such as isophoronediamine, 1,3-bisaminomethylcyclohexane, diaminocyclohexanes, and bis(4-aminocyclohexyl)methane; alkanolamines, such as ethanolamine; poly(oxypropylene)diamines; and meta-xylene diamine.

The epoxy resin comprising $C_{15}$ alkenyl group containing diglycidyl ether made in accordance with the methods of the present invention provides high solids two component coating compositions suitable for concrete and steel coating. Such coating compositions may be formed by mixing the epoxy resin component and a hardener, e.g. an amine or a polyamine, second component.

The polyamine adducts of the low viscosity $C_{15}$ alkenyl group containing diglycidyl ether epoxy resin of the present invention and, preferably, such epoxy resins that are substantially free of oligomers from side chain coupling reactions of alkenyl groups provide high solids two component coating compositions suitable for concrete and steel coating. Such coating compositions may be formed by mixing the inventive amine adduct and an epoxy resin, including the $C_{15}$ alkenyl group containing diglycidyl ether compositions made in accordance with the methods of the present invention and conventional epoxy resins, e.g. an epoxy resin based on bisphenol A, bisphenol F or a novolac, second component.

The two component coating compositions may have a solids content of 85 wt.% solids or higher, preferably, 95 wt.% or higher, e.g. 100 wt.% solids wherein the compositions can be easily applied by conventional spray, roll, dip, and brush methods.

The coating compositions of the present invention have initial ambient temperature viscosities, i.e. on mixing, that enable them to be sprayed even at very high solids contents of above 85 wt.%, or above 95 wt.%, or even above 97 wt.%. Such initial coating composition viscosities may range, for example, from 50 to 3000 cPs, preferably, 1000 cPs or less, or, preferably, 100 cPs or more, or, more preferably, 800 cPs or less.

If desired, the high solids coating compositions of the present invention may include up to 15 wt. % solvent or diluent, preferably, up to 10 wt. %, or, more preferably, up to 5 wt. %. Suitable solvents and diluents may include, for example, methylethylketone (MEK), xylene, toluene, aromatic hydrocarbons and petroleum distillates, butanol, and benzyl alcohol. Suitable reactive diluents may include, for example, cresol glycidyl ether, butyl glycidyl ether and $C_{12}$-$C_{14}$ aliphatic glycidyl ether, and diglycidyl ethers such butanediol diglycidyl ether, hexanediol diglycidyl ether, cyclohexanedimethanol diglycidyl ether, and triglycidyl ethers such as trimethylolpropane triglycidyl ether and glycerol triglycidyl ether.

Where the epoxy resins of the present invention are used as diluents, compositions comprising them may further comprise any conventional epoxy resins, such as bisphenol A or F epoxy resins, phenolic epoxy resins, polyphenolic epoxy resins, novolac epoxy resins and cresol epoxy resins, as well as mixtures thereof. Such compositions may have higher initial viscosities than two component mixtures containing only hardener and the epoxy resin comprising a $C_{15}$ alkenyl group containing diglycidyl ether of the present invention. In such compositions, the total initial viscosity of a coating composition would be lowered by including the low viscosity epoxy resin of the present invention.

A suitable second component or hardener may be any conventional hardener for epoxy resins. Conventional hardeners may be, for example, any amine or mercaptan with at least two epoxy reactive hydrogen atoms per molecule, anhydrides, or phenolics. Preferably, the hardener is an amine where the nitrogen atoms are linked by divalent hydrocarbon groups that contain at least 2 carbon atoms per subunit, such as aliphatic, cycloaliphatic or aromatic groups. Preferably, polyamines contain from 2 to 6 amine nitrogen atoms per molecule, from 2 to 8 amine hydrogen atoms per molecule, and 2 to about 50 carbon atoms.

Examples of suitable polyamines include aliphatic polyamines such as, for example, ethylene diamine, diethylenetriamine, triethylenetetramine; cycloaliphatic polyamines such as, for example, isophoronediamine, 1,3-bis(aminomethyl)cyclohexane, isomeric mixtures of bis(4-aminocyclohexyl)methanes; bicyclic imines, such as, for example, 3-azabicyclo[3.3.1]non-2-ene; bicyclic diamines, such as, for example, 3-azabicyclo[3.3.1]nonan-2-amine; heterocyclic diamines such as, for example, 3,4-diaminofuran, aminoethylpiperazine, and piperazine; polyamines containing amide linkages derived from "dimer acids" (dimerized fatty acids) which are produced by condensing the dimer acids with ammonia and then optionally hydrogenating; adducts of suitable amines with epoxy resins, epichlorohydrin, acrylonitrile, acrylic monomers, ethylene oxide, and the like, such as, for example, an adduct of isophoronediamine with a diglycidyl ether of a dihydric phenol; aromatic polyamines such as, for example, 4,4'-methylenedianiline, 1,3-phenylenediamine and 3,5- diethyl-2,4-toluenediamine; amidoamines such as, for example, condensates of fatty acids with diethylenetriamine or triethylenetetramine; polyamides such as, for example, condensates of dimer acids with diethylenetriamine, triethylenetetramine; oligo(propylene oxide)diamine; and Mannich bases, such as, for example, the condensation products of a phenol, formaldehyde, and a polyamine or phenalkamines. Mixtures of more than one diamine and/or polyamine can also be used. Primary monoamines may be included as chain extending agents.

Other curing agents and accelerators that may be used in the second component with a hardener as described above may include quaternary ammonium and phosphonium salts, such as, for example, tetraethylammonium chloride; phosphines; nitrate salts, such as, for example, calcium nitrate; and phosphites, such as, for example, triphenylphosphite or combinations thereof as described in U.S. Pat. Nos. 5,208,317, 5,109,099 and 4,981,926, phenolic compounds such as t-butylphenol, bisphenol A, salicylic acid and aminophenols such as 2,4,6-tris(dimethylamionomethyl) phenol.

The stoichiometric ratio of epoxy resin in the epoxy component to the hardener in the second component of the coating compositions may range from 0.5:1 to 1:0.5, preferably, from 0.7:1 to 1:0.7, or, more, preferably, from 0.8:1 to 1:0.8, or, most preferably, 0.90:1 to 1:0.90.

The coating compositions of the present invention may be clearcoats, wherein they have no pigments or may include pigments or fillers that do not alter clarity, such as subcritical amounts of pigments having a refractive index of less than 1.7, e.g. silica, talc, calcium carbonate or alumina.

The coating compositions of the present invention can be pigmented/filled using additives including pigments, which may be organic or inorganic and may functionally contribute to opacity, e.g. titanium dioxide or hollow core or void containing polymer pigments, and color, e.g. iron oxides, micas, aluminum flakes and glass flakes, silica pigments, or organic pigments, such as phthalocyanines, and corrosion protection, e.g. zinc, phosphates, molybdates, chromates, vanadates, cerates, in addition to durability and hardness such as silicates. Generally, when pigments are included in the coating compositions, the weight ratio of pigment to the total solid of epoxy resin and hardener may range from 0.1:1 to 5:1, preferably, up to 3:1.

The coating compositions of the present invention may include other conventional additives in conventional amounts, including, for example, rheology modifiers, dispersants, silicones or wetting agents, adhesion promoters, or flow and leveling agents.

The coating compositions of the present invention may be applied to substrates, preferably, by spraying them, to form a coating layer. Suitable substrates may include composites, metals such as steel and aluminum, and concrete. The coating compositions enable greater application flexibility, as they have a low initial viscosity and so can be pumped or conveyed greater distances to reach higher or more difficult to reach objects and substrates. Preferably, the methods are field applications, meaning that they take place where the substrate is located, i.e. the applying is carried out in the field and not in a factory adapted for coating applications.

The coating compositions of the present invention are suitable for use in factory and field applications such as coatings for use on substrates such as concrete, metal, machinery, heavy mass parts, ships, buildings under construction, bridges, tanks, anticorrosive applications e.g. pipe coatings, and flooring and maintenance coating applications. Examples of substrates may include metal structures, like water towers or bridges, pipes, tanks, ships, heavy mass parts, such as girders, machinery, such as heavy equipment, bridges, concrete structures, and buildings.

In one example of the methods useful to make protective finishes, the coating compositions of the present invention may be used as a primer, and the methods further comprise applying additional layers over the primer.

The following examples are used to illustrate the present invention without limiting it to those examples. Unless otherwise indicated, all temperatures are ambient temperatures and all pressures are 1 atmosphere.

The following standard abbreviations are used in the Examples and Comparative Examples: "MGE" stands for monoglycidyl ether(s), "DGE" stands for diglycidyl ether(s), "DETA" stands for diethylenetriamine, "GC" stands for gas chromatography (chromatographic); "MS" stands for mass spectrometry (spectrometric); "EEW" stands for epoxide equivalent weight; "DI" stands for deionized; "eq" stands for equivalent(s); "wt" stands for weight(s); "vol" stands for volume(s); "min" stands for minute(s); "hr" stands for hour(s); "g" stands for gram(s); "mL" stands for milliliter(s); "L" stands for liter(s); "LPM" stands for liter(s) per minute; "$\mu$m" stands for micrometer(s); "mm" stands for millimeter(s); "m" stands for meter(s); "J" stands for joule(s); and "cP" stands for centipoise.

The analytical equipment and methods used in the examples are, as follows:

Differential Scanning Calorimetry (DSC): A DSC 2910 Modulated DSC (TA Instruments, New Castle, DE) was employed, using a heating rate of 7°C per min with the temperature range given in the respective examples under a stream of nitrogen flowing at 35 cm$^3$/min. The sample was contained in an aluminum pan and loosely covered (not sealed) with an aluminum lid. The sample weight tested is given with the results obtained. For analysis of Tg, the aforementioned parameters were again employed. The sample was contained in an open aluminum pan.

Viscosity and Density: Viscosity and density were determined at the indicated analyte solids content on a Stabinger Viscometer (Model SVM 3000, Anton Paar(Ashland, VA) at 25°C.

Percent Epoxide / Epoxide Equivalent Weight Analysis: The titration method used to determine percent epoxide in the various epoxy resins can be found in Jay, R.R., "Direct Titration of Epoxy Compounds and Aziridines", Analytical Chemistry, 36, 3, 667-668 (March, 1964). In the present method, a carefully weighed sample (sample weight ranges from 0.17 - 0.25 g) was dissolved in dichloromethane (15 mL) followed by the addition of tetraethylammonium bromide solution in acetic acid (15 mL). The resultant solution treated with 3 drops of crystal violet indicator (0.1 % wt/vol in acetic acid) was titrated with 0.1 N perchloric acid in acetic acid on a Metrohm™ 665 Dosimat™ titrator (Brinkmann, Riverview, FL). Titration of a blank consisting of dichloromethane (15 mL) and tetraethylammonium bromide solution in acetic acid (15 mL) provided correction for solvent background. Percent epoxide and EEW were calculated using the following equations:

$$\text{\% Epoxide} = \frac{[(\text{mL titrated sample}) - (\text{mL titrated blank})]\ (0.4303)}{(\text{g sample titrated})}$$

$$\text{EEW} = \frac{43.03}{\text{\% epoxide}}$$

Gas Chromatogaphic (GC) Analysis: In the general method, a Hewlett Packard 5890 Series II Plus gas chromatograph was employed using a DB-1 capillary column (61.4 m by 0.25 mm with a 0.25 $\mu$m film thickness, Agilent (Santa Clara, CA). The column was maintained in the chromatograph oven at a 50 °C initial temperature. Both the injector inlet and flame ionization detector were maintained at 300 °C. Helium carrier gas flow through the column was maintained at 1.1 mL per min. GC analyses in area % are not a quantitative measure of any given component.

Samples for GC analysis were prepared by collection of a 0.5 mL aliquot of the slurry product from the epoxidation and addition to a vial containing 1 mL of acetonitrile. After shaking to mix, a portion of the slurry in acetonitrile was loaded into a 1 mL syringe (Norm-Ject, all polypropylene / polyethylene, Henke Sass Wolf GmbH, (Tuttlingen, Germany) and passed through a syringe filter (Acrodisc CR 13 with 0.2 $\mu$m PTFE membrane, Pall Corporation, Gelman Laboratories, Port Washington, NY) to remove any insoluble debris.

Example 1 - Production of Cardanol / Cardol Epoxy Resin

A mixture nominally containing 55.6 % cardanol and 44.4 % cardol was obtained as a product from the processing cashew nut shell liquid. An average hydroxyl equivalent weight of 224.3 was calculated. GC analysis demonstrated the presence of 54.63 area % cardanol and 40.07 area % cardol with the balance as seven minor components (0.12, 0.09, 0.17, 0.27, 3.57, 0.58, 0.50 area %, respectively).

Example 1A. Epoxidation Reaction A 5 L, 4 neck, glass, round bottom reactor was charged with cardol and cardanol mixture (750.0 g, 3.7 hydroxyl eq), epichlorohydrin (1710.60 g, 18.48 moles, 5:1 epichlorohydrin:hydroxyl eq), isopropanol (921.1 g, 35 wt % of epichlorohydrin used), and DI water (148.8 g, 6 wt % of epichlorohydrin used) in the indicated order. The reactor was additionally equipped with a condenser (maintained at 0 °C), a thermometer, a Claisen adaptor, an overhead nitrogen inlet (1 LPM $N_2$ used), and a stirrer assembly, which has a poly(tetrafluoroethylene) paddle, glass shaft, variable speed motor. A controller monitored the temperature registered on the thermometer in the reactor and provided heating via the heating mantle placed under the reactor as well as cooling delivered by a pair of fans positioned on the reactor exterior. Sodium hydroxide (133.05 g, 3.33 moles) dissolved in DI water (532.2 g) for the initial addition was added to a side arm vented addition funnel, sealed with a ground glass stopper, then attached to the reactor. Stirring and heating commenced to give a 52 °C solution followed by dropwise addition of the aqueous sodium hydroxide solution. The reaction temperature was maintained at 52 °C during the 2.25 hr aqueous sodium hydroxide addition time via cooling of the reactor exterior from the fans as needed. After 20 min of postreaction at 52 °C, stirring and heating ceased, and the reactor contents were added to a pair of 2 L separatory funnels. The aqueous phase and a minor amount of dark colored insoluble material were drained off and discarded as waste and the remaining organic layer added back into the reactor.

Stirring and heating of the 44 °C solution resumed. Sodium hydroxide (59.13 g, 1.478 moles) dissolved in DI water (236.5 g) was added to a side arm vented addition funnel, sealed with a ground glass stopper, then attached to the reactor. Stirring and heating commenced to reestablish a 52 °C solution followed by dropwise addition of aqueous sodium hydroxide solution. Reaction temperature was maintained at 52 °C during the 1 hr aqueous sodium hydroxide addition time via cooling of the reactor exterior from the fans as needed. After 20 min of postreaction at 52 °C, stirring and heating ceased, and the reactor contents were added to a pair of 2 L separatory funnels. The aqueous phase and a minor amount of dark colored insoluble material were drained off and discarded as waste and the remaining organic layer added back into the reactor.

Stirring and heating of the 40 °C solution resumed. Sodium hydroxide (14.78 g, 0.37 moles) dissolved in DI water (59.1 g) was added to a side arm vented addition funnel, sealed with a ground glass stopper, then attached to the reactor. Stirring and heating commenced to reestablish a 52 °C solution followed by dropwise addition of the aqueous sodium hydroxide solution. Reaction temperature was maintained at 52 °C during the 15 min aqueous sodium hydroxide addition time via cooling of the reactor exterior from the fans as needed. After 20 min of postreaction at 52 °C, stirring and heating ceased, and the reactor contents were equally split into a pair of 2 L separatory funnels. The aqueous phase was drained off and discarded as waste. The contents of each separatory funnel were washed by vigorously shaking with 400 mL of DI water. The washed product was allowed to settle for <30 min to resolve the aqueous and organic phases then the aqueous phase was removed and discarded as waste. Second and third washes were completed using the aforementioned method.

Rotary evaporation of the organic phase using a maximum oil bath temperature of 110 °C to a final vacuum of 4.7 mm of Hg removed the bulk of the volatiles. A total of 911.52 g of amber colored, slightly hazy liquid was recovered after completion of the rotary evaporation. GC analysis after normalization to remove solvent (acetonitrile) revealed the presence of 55.33 area % monoglycidyl ethers of cardanol, 39.74 area % diglycidyl ethers of cardol, 0.75 area % as four minor components with retention times lower than that of the monoglycidyl ethers of cardanol, and 4.18 area % as four minor components with retention times between the monoglycidyl ethers of cardanol and the diglycidyl ethers of cardol. Epoxide titration indicated an EEW of 287.55.

Example 1B: Fractional Vacuum Distillation with in situ Process Inhibitor A portion (200.0 g) of the product from the rotary evaporation of A. above and 2,6-di-tertiary-butyl-4-methylphenol (2.0 g) were added to a 250 mL, 3 neck, glass, round bottom reactor equipped with magnetic stirring and a thermometer for monitoring the pot temperature. A one piece integral open glass tube distillation column with distillation head was attached to the reactor. The open glass tube comprising the distillation column was 9 cm tall by 1.9 cm inside diameter. The distillation head was equipped with an overhead thermometer, air cooled condenser, a receiver and a vacuum takeoff. A vacuum pump was employed along with a liquid nitrogen trap and an in-line digital thermal conductivity vacuum gauge. Stirring and heating of the distillation pot to 163 °C commenced followed by application of full vacuum then progressively increased heating using a thermostatically controlled heating mantle. The heating from 163 °C to a maximum of 250 °C under vacuum ranging from 0.047 to 0.107 Torr was completed over a 325 min. During the distillation, a single distillation cut (132.24 g) was taken to remove all residual minor components boiling below the cardanol monoglycidyl ethers (including part of the 2,6-di-tertiary-butyl-4-methylphenol process inhibitor), cardanol monoglycidyl ethers, and components with retention times between the monoglycidyl ethers of cardanol and the diglycidyl ethers of cardol while minimizing removal of diglycidyl ethers of cardol. GC analysis of the product (69.21 g) remaining in the distillation pot revealed the presence of 0.21 area % cardanol monoglycidyl ethers, 0.51 area % of a minor component with retention time between the monoglycidyl ethers of cardanol and the diglycidyl ethers of cardol, and 99.28 area % diglycidyl ethers of cardol. Epoxide titration indicated an EEW of 274.27. Viscosity of the amber colored, transparent liquid at 25 °C was 642.5 cP and density was 1.0352.

Example 2 (Utility)- Preparation and Curing of a Thermosettable Composition of the Diglycidyl Ethers of Cardol and Diethylenetriamine

A portion (11.54 g, 0.042 epoxide eq) of the diglycidyl ethers of cardol (distillation bottoms) from Example 1 B. and DETA (0.87 g, 0.042 N-H eq) were added to a glass bottle and vigorously stirred together. A portion (8.4 mg) of the homogeneous solution was removed for DSC analysis. An exotherm attributed to curing was observed with a 27.4 °C onset, 102.2 °C maximum, and a 176.1 °C endpoint accompanied by an enthalpy of 316 J/g. The cured product recovered from the DSC analysis was an amber colored tack-free, transparent, rigid solid.

Example 3 (Utility)- Preparation of Clear, Unfilled Casting from a Thermosettable Composition of the Diglycidyl Ethers of Cardol and Diethylenetriamine and Analysis of Glass Transition Temperature

The remaining portion of the diglycidyl ethers of cardol (distillation bottoms) and DETA blend from Example 2 was added to an aluminum dish and cured in an oven using the following schedule: 1 hr at 75 °C, 1 hr at 100 °C, 1 hr at 125 °C, and 1 hr at 150 °C. A portion (22.7 mg) of the transparent, rigid, tack-free, amber colored casting was removed for DSC analysis from 0 °C to 200 °C. A Tg of 60.9 °C was observed, with no indication of further curing or exothermic decomposition observed up to the 200 °C DSC analysis temperature. A second scan using the aforementioned conditions revealed a 60.3 °C Tg. This scan indicates excellent thermal stability.

Example 4 - Fractional Vacuum Distillation with in situ Process Inhibitor and Partial Oligomerization

A portion (90.56 g) of the product from the rotary evaporation of Example 1 A. above and 2,6-di-tertiary-butyl-4-methylphenol (0.905 g) were added to a 100 mL, 3 neck, glass, round bottom reactor equipped with magnetic stirring and a thermometer for monitoring the pot temperature. The one piece integral open glass tube distillation column with distillation head used in Example 1 B. was attached to the reactor. Stirring and heating of the distillation pot to 167 °C commenced followed by application of full vacuum then progressively increased heating using a thermostatically controlled heating mantle. The heating from 167 °C to a maximum of 246 °C under vacuum ranging from 0.042 to 0.092 Torr was completed over 278 min. During the distillation, a single distillation cut (67.90 g) was taken to remove all residual minor components boiling below the cardanol monoglycidyl ethers (including the 2,6-di-tertiary-butyl-4-methylphenol process inhibitor), cardanol monoglycidyl ethers, and components with retention times between the monoglycidyl ethers of cardanol and the diglycidyl ethers of cardol while minimizing removal of diglycidyl ethers of cardol. GC analysis of the product (23.06 g) remaining in the distillation pot revealed no detectable cardanol monoglycidyl ethers, 90.99 area % diglycidyl ethers of cardol and 9.01 area % multiple minor components (oligomers). Epoxide titration indicated an EEW of 306.48. Viscosity of the amber colored, transparent liquid at 25 °C was 1347.4 cP and density was 1.0406.

Example 5 - Preparation and Curing of a Thermosettable Composition of the Diglycidyl Ethers of Cardol Containing Oligomers and Diethylenetriamine

A portion (12.10 g, 0.0395 epoxide eq) of the diglycidyl ethers of cardol containing oligomers (distillation bottoms) from Example 4 and DETA (0.814 g, 0.0395 N-H eq) were added to a glass bottle and vigorously stirred together. A portion (9.2 mg) of the homogeneous solution was removed for DSC analysis. An exotherm attributed to curing was observed with a 35.93 °C onset, 100.85 °C maximum, and a 198.45 °C endpoint accompanied by an enthalpy of 283 J/g. The cured product recovered from the DSC analysis was an amber colored, tack-free, transparent, rigid solid.

Example 6 - Preparation of Clear, Unfilled Casting from a Thermosettable Composition of the Diglycidyl Ethers of Cardol Containing Oligomers and Diethylenetriamine and Analysis of Glass Transition Temperature

The remaining portion of the diglycidyl ethers of cardol containing oligomers (distillation bottoms) and DETA blend from Example 5 was added to an aluminum dish and cured in an oven using the following schedule: 1 hr at 75 °C, 1 hr at 100 °C, 1 hr at 125 °C, and 1 hr at 150 °C. A portion (34.5 mg) of the transparent, rigid, tack-free, amber colored casting was removed for DSC analysis from 0 °C to 200 °C. A Tg of 51.0 °C was observed, with no indication of further curing or exothermic decomposition observed up to the 200 °C DSC analysis temperature. A second scanning using the aforementioned conditions revealed a 52.2 °C Tg.

Comparative Example 7 - Production of Cardanol/ Cardol Epoxy Resin

The epoxidation of Example 1A was repeated. Rotary evaporation of the organic phase using a maximum oil bath temperature of 110 °C to a final vacuum of 4.0 mm of Hg removed the bulk of the volatiles. A total of 912.21 g of amber colored, slightly hazy liquid was recovered after completion of the rotary evaporation. GC analysis after normalization to remove solvent (acetonitrile) revealed the presence of 56.08 area % monoglycidyl ethers of cardanol, 39.32 area % diglycidyl ethers of cardol, 0.47 area % as four minor components with retention times lower than that of the monoglycidyl ethers of cardanol, and 4.13 area % as four minor components with retention times between the monoglycidyl ethers of cardanol and the diglycidyl ethers of cardol. Epoxide titration indicated an EEW of 288.81. Viscosity of the amber colored, transparent liquid at 25 °C was 58.9 cP and density was 0.9902.

Comparative Example A - Preparation and Curing of a Thermosettable Blend of Cardanol / Cardol Epoxy Resin and Diethylenetriamine

A portion (12.2390 g, 0.04238 epoxide eq) of the cardanol / cardol epoxy resin from Comparative Example 7 and DETA (0.8742 g, 0.04238 N-H eq) were added to a glass bottle and vigorously stirred together. A portion (11.3 mg) of the homogeneous solution was removed for DSC analysis. An exotherm attributed to curing was observed with a 52.21 °C onset, 108.58 °C maximum, and a 207.65 °C endpoint accompanied by an enthalpy of 245 J/g. The cured product recovered from the DSC analysis was amber colored, transparent and tacky.

Comparative Example B - Preparation of Clear, Unfilled Casting from a Thermosettable Blend of Cardanol / Cardol Epoxy Resin and Diethylenetriamine

The remaining portion of the of the cardanol / cardol epoxy resin and DETA blend from Comparative Example A was added to an aluminum dish and cured in an oven using the following schedule: 1 hr at 75 °C, 1 hr at 100 °C, 1 hr at 125 °C, and 1 hr at 150 °C. The resultant amber colored, transparent cured product was tacky and gelatinous.

The Comparative Examples A and B show that a high level of cardanol MGE's ($C_{15}$ alkenyl group containing monoglycidyl ethers) in an epoxy resin composition produce a poor product (tacky surface, etc.) not suitable for coatings.

Comparative Example 8 - Thermal Treatment and Fractional Vacuum Distillation to Produce a Cardanol / Cardol Epoxy Resin with Oligomers

A portion (750.0 g) of the product from the rotary evaporation of Example 7 was added to a 1 L, 3 neck, glass, round bottom reactor equipped with magnetic stirring and a thermometer for monitoring the pot temperature. A vacuum jacketed Vigreux column with distillation head was attached to the reactor. The distillation column was 61 cm tall by 2 cm inside diameter. The distillation head was equipped with an overhead thermometer, air cooled condenser, a receiver and a vacuum takeoff. The product was initially heated to 175 °C and then placed under vacuum and slowly heated to a maximum temperature of 252 °C over 7.7 hr. During this time, the vacuum ranged from 0.048 to 0.383 Torr and induced refluxing of the epoxy resin without distillation. After cooling under vacuum to 25 °C, the Vigreux column and distillation head were removed and replaced by a one piece integral, vacuum jacketed Vigreux distillation column with distillation head. The distillation column was 8 inches tall by 0.75 inch inside diameter. The distillation head was equipped with an overhead thermometer, air cooled condenser, a receiver and a vacuum takeoff. A vacuum pump was employed along with a liquid nitrogen trap and an in-line digital thermal conductivity vacuum gauge. Stirring and heating of the distillation pot to 192 °C commenced followed by application of full vacuum then progressively increased heating using a thermostatically controlled heating mantle. The heating from 192 °C to a maximum of 260 °C under vacuum ranging from 0.094 to 0.365 Torr was completed over 7.45 hr. During the distillation, distillation cuts (288.87 g total weight) were taken to remove residual minor components boiling below the cardanol monoglycidyl ethers and a portion of the cardanol monoglycidyl ethers, while minimizing removal of diglycidyl ethers of cardol. GC analysis of the product (458.04 g) remaining in the distillation pot revealed the presence of 14.27 area % cardanol monoglycidyl ethers, 7.63 area % of minor components with retention times between the monoglycidyl ethers of cardanol and the diglycidyl ethers of cardol, 60.02 area % diglycidyl ethers of cardol, and 18.08 area % oligomers. Epoxide titration indicated an EEW of 310.6. Viscosity of the amber colored, transparent liquid at 25 °C was 2273.5 cP and density was 1.0385.

Example 9 - Fractional Vacuum Distillation with in situ Process Inhibitor to Produce a Cardanol / Cardol Epoxy Resin (Oligomer-free)

A portion (73.73 g) of the product from the rotary evaporation of Comparative Example 7 and 2,6-di-tertiary-butyl-4-methylphenol (0.74 g) were added to a 100 mL, 3 neck, glass, round bottom reactor equipped with magnetic stirring and a thermometer for monitoring the pot temperature. The one piece integral open glass tube distillation column with distillation head used in Example 1 B. was attached to the reactor. Stirring and heating of the distillation pot to 175 °C commenced followed by application of full vacuum then progressively increased heating using a thermostatically controlled heating mantle. The heating from 175 °C to a maximum of 227 °C under vacuum ranging from 0.034 to 0.129 Torr was completed over 8.2 hr. During the distillation, a single distillation cut (36.38 g) was taken to remove all residual minor components boiling below the cardanol monoglycidyl ethers (including part of the 2,6-di-tertiary-butyl-4-methylphenol process inhibitor) and a portion of the cardanol monoglycidyl ethers, while minimizing removal of diglycidyl ethers of cardol. GC analysis of the product (37.57 g) remaining in the distillation pot revealed 11.73 area % cardanol monoglycidyl ethers, 2.62 area % of minor components with retention times between the monoglycidyl ethers of cardanol and the diglycidyl ethers of cardol, 85.65 area % diglycidyl ethers of cardol, and no detectable oligomers. Epoxide titration indicated an EEW of 264.26. Viscosity of the amber colored, transparent liquid at 25 °C was 256.3 cP and density was 1.0235.

Example 10 - Preparation and Curing of a Thermosettable Composition of a Cardanol / Cardol Epoxy Resin (Oligomer-free) and Diethylenetriamine

A portion (12.0060 g, 0.04543 epoxide eq) of the cardanol / cardol epoxy resin of cardol (distillation bottoms) from Example 9 and DETA (0.9372 g, 0.04543 N-H eq) were added to a glass bottle and vigorously stirred together. A portion (11.3 mg) of the homogeneous solution was removed for DSC analysis. An exotherm attributed to curing was observed with a 40.71 °C onset, 103.86 °C maximum, and a 194.62 °C endpoint accompanied by an enthalpy of 295 J/g. The cured product recovered from the DSC analysis was an amber colored, tack-free, transparent, rigid solid.

Example 11 - Preparation of Clear, Unfilled Casting from a Thermosettable Composition of a Cardanol / Cardol Epoxy Resin (Oligomer-free) and Diethylenetriamine and Analysis of Glass Transition Temperature

The remaining portion of the cardanol / cardol epoxy resin (distillation bottoms) and DETA blend from Example 12 was added to an aluminum dish and cured in an oven using the following schedule: 1 hr at 75 °C, 1 hr at 100 °C, 1 hr at 125 °C, and 1 hr at 150 °C. A portion (32.7 mg) of the transparent, rigid, tack-free, amber colored casting was removed for DSC analysis from 0 °C to 200 °C. A Tg of 52.0 °C was observed, with no indication of further curing or exothermic decomposition observed up to the 200 °C DSC analysis temperature. A second scanning using the aforementioned conditions revealed a 52.0 °C Tg. A third scanning from 0 °C to 250 °C gave a Tg of 52.6 °C with no indication of further curing or exothermic decomposition observed up to the 250 °C DSC analysis temperature. Fourth and fifth scannings using the aforementioned conditions gave a 56.1 °C and a 58.4 °C Tg, respectively.

Example 12 - Water Absorption Testing of Clear, Unfilled Casting from a Thermosettable Composition of a Cardanol / Cardol Epoxy Resin (Oligomer-free) and Diethylenetriamine

A portion of the clear, unfilled casting from Example 11 was weighed, added to a polyethylene bottle containing DI water (100 g) and held at 24 °C. The casting was removed periodically, blotted dry and then weighed. The weights were used to calculate the percent water absorption given in Table I, below.

Comparative Example 13 - Water Absorption Testing of Clear, Unfilled Casting from a Thermosettable Composition of a Bisphenol A Epoxy Resin and Diethylenetriamine

A clear, unfilled casting was prepared by mixing epoxy resin of bisphenol A (D.E.R.™ 332, Dow Chemical, Midland, MI) (10.7490 g, 0.06279 epoxide equivalent) and DETA (1.2952 , 0.06279 -NH equivalent) followed by curing in an aluminum dish in an oven using the following schedule: 1 hr at 75 °C, 1 hr at 100 °C, 1 hr at 125 °C, and 1 hr at 150 °C. A portion of the clear, unfilled casting was weighed, added to a polyethylene bottle containing DI water (100 g) and held at 24 °C. The casting was removed periodically, blotted dry and then weighed. The weights were used to calculate the percent water absorption given in Table I, below.

Example 14 - Water Absorption Testing of Clear, Unfilled Casting from a Thermosettable Composition of a Cardanol / Cardol Epoxy Resin with Oligomers and Diethylenetriamine

A portion of the clear, unfilled casting from Example 10 was weighed, added to a polyethylene bottle containing DI water (100 g) and held at 24 °C. The casting was removed periodically, blotted dry and then weighed. The weights were used to calculate the percent water absorption given in Table I.

As shown in Table 1, below, the inventive compositions exhibit lower water absorption then the comparative conventional epoxy resins, thereby enabling them to retain a higher Tg and its corresponding chemical resistance in the field.

Table I: Percent Water Absorption

| Exposure Time (hr) | Example 12 (% wt. water absorbance) | Example 14 (% wt. water absorbance) | Comparative Example 13 (% wt. water absorbance) |
|---|---|---|---|
| 4 | 0.05 | 0.06 | 0.07 |
| 24 | 0.16 | 0.13 | 0.16 |
| 48 | 0.19 | 0.22 | 0.24 |
| 145 | 0.35 | 0.39 | 0.40 |

Example 15 - Water Desorption Testing of Clear, Unfilled Casting from a Thermosettable Blend of a Cardanol / Cardol Epoxy Resin (Oligomer-free) and Diethylenetriamine The clear unfilled casting from Example 12 was removed from DI water after 672 hr at 24°C, blotted dry, weighed and then held at 24 °C and 50 % relative humidity. The casting was periodically weighed. The weights were used to calculate the percent water desorption given in Table II, below.

Example 16 - Water Desorption Testing of Clear, Unfilled Casting from a Thermosettable Blend of a Cardanol / Cardol Epoxy Resin with Oligomers and Diethylenetriamine The clear unfilled casting from Example 14 was removed from DI water after 672 hr at 24°C and tested using the method of Example 15. Percent water desorption is given in Table II, below.

Comparative Example 17 - Water Absorption Testing of Clear, Unfilled Casting from a Thermosettable Blend of a Bisphenol A Epoxy Resin and Diethylenetriamine

The clear unfilled casting from Comparative Example 13 was removed from DI water after 672 hr at 24°C and tested using the method of Example 15. Percent water desorption is given in Table II, below.

As shown in Table II, below, the compositions of the present invention shed water more quickly in the field than do conventional epoxy resins, especially after 24 hours, and thereby retain a higher Tg and its corresponding chemical resistance properties.

Table II: Percent Water Desorption

| Exposure Time (hr) | Example 15 (% wt. water desorbance) | Example 16 (% wt. water desorbance) | Comparative Example 17 (% wt. water desorbance) |
|---|---|---|---|
| 5 | 0.083 | 0.073 | 0.086 |
| 8 | 0.098 | 0.092 | 0.096 |
| 24 | 0.14 | 0.15 | 0.12 |
| 48 | 0.18 | 0.20 | 0.14 |
| 72 | 0.20 | 0.24 | 0.15 |
| 144 | 0.25 | 0.30 | 0.17 |
| 248 | 0.28 | 0.35 | 0.18 |
| 368 | 0.30 | 0.39 | 0.18 |

Example 18 - Preparation and Characterization of the Diethylenetriamine Adduct of the Diglycidyl Ether of Cardol

A 1 L 3 neck, glass, round bottom, reactor was charged under nitrogen with DETA (103.1 g, 1 mole, 5 amine hydrogen eq). The DETA (Sigma-Aldrich Chemical Company, St. Louis, MO) had a purity specification of 99 %. The reactor was additionally equipped with a condenser (maintained at 0 °C), a thermometer, a Claisen adaptor, an overhead nitrogen inlet (1 LPM $N_2$ used), and magnetic stirring. A portion (13.71 g, 0.05 epoxide eq) of DGE of cardol having an EEW of 274.27 from Example 1 B was added to a side arm vented addition funnel, and then attached to the reactor. Stirring and heating using a thermostatically controlled heating mantle commenced to give a 40 °C solution. Dropwise addition of the DGE of cardol commenced while maintaining the reaction temperature at 40 °C. After 3 hrs the dropwise addition was completed. The resultant light amber colored reaction mixture was stirred and maintained at 40°C for the next 38.7 hrs followed by rotary evaporation at 80 °C to remove the bulk of the excess DETA. Additional rotary evaporation was completed using a maximum 125 °C oil bath temperatures to a vacuum of 0.48 mm Hg. A transparent, light amber colored, liquid adduct product was recovered from the rotary evaporation (20.06 g). GC analysis of an aliquot of the adduct product revealed that complete reaction of all DGE of cardol had occurred. Titration of portions of the adduct indicated an average AHEW of 78.9. AHEW is measured in the manner stated above.

Example 19 - Preparation and Curing of Thermosettable Composition of the Diethylenetriamine Adduct of the Diglycidyl Ether of Cardol and Diglycidyl Ether of Cardol

DGE of cardol (7.18 g, 0.026 epoxide eq) from Example 1 B. and DETA adduct of cardol epoxy from Example 18 (2.06 g, 0.026 N-H eq) were added to a glass vial and vigorously stirred together. A portion (8.3 mg) of the homogeneous light amber colored solution was immediately removed for DSC analysis. An exotherm attributed to curing was observed with a 33.09 °C onset, 93.72 °C maximum, and a 192.43 °C endpoint accompanied by an enthalpy of 254.0 J/g. The enthalpy per gram is about 20% below the enthalpy of a mixture of the same cardol diglycidyl ether mixed with unmodified DETA in the same equivalent amounts, thereby showing greatly reduced cure energy. The cured product recovered from the DSC analysis was a transparent, rigid, tack-free, amber colored solid.

Example 20 - Preparation of Clear, Unfilled Casting of Thermosettable Composition of the Diethylenetriamine Adduct of Cardol and Diglycidyl Ether of Cardol and Analysis of Glass Transition Temperature

The remaining portion of a curable blend from Example 19 was added to an aluminum dish and cured in an oven for 1 hr at 70 °C, 1 hr at 100 °C, 1 hr at 125 °C and 1 hr at 150 °C. A portion (34.5 mg) of the transparent, rigid, tack-free, amber colored casting was removed for DSC analysis. A Tg of 60.13 °C was observed with no indication of further curing or exothermic decomposition observed up to the 200 °C DSC analysis temperature. A second scanning using the aforementioned conditions revealed a 59.28 °C Tg. A third scanning was completed with an increase in the final temperature to 250 °C giving a Tg of 62.43 °C with no indication of further curing or exothermic decomposition observed up to the 250 °C DSC analysis temperature. A fourth and fifth scannings using the aforementioned conditions gave a Tg of 63.81 °C and 63.05 °C, respectively.

Table 3: Cure Profiles

| Designation | Onset (°C) | Maximum (°C) | End (°C) | Enthalpy (J/g) |
|---|---|---|---|---|
| Example 19 | 33.1 | 93.7 | 192.4 | 254 |
| Example 2 | 27.4 | 102.2 | 176.1 | 316 |

The inventive DETA adduct of cardol diglycidyl ether of Example 19 versus a mixture of DETA to cure the same cardol diglycidyl ether (Example 2) illustrates advantages in cure profile for the inventive adducts, notably reduced enthalpic release of curing energy while maintaining a rapid, low temperature cure exhibited by the adduct composition.
The inventive DETA adduct of cardol diglycidyl ether of Example 20 when compared to the same components in a mixture DETA and cardol diglycidyl ether in Example 3 provides a 3 °C increase in Tg after cycling versus no or very little change in the Tg, thereby evidencing the durability of cured products containing the adduct.

**Claims**

1. A method of forming a low viscosity epoxy resin composition comprising distilling an epoxy resin mixture of one or more $C_{15}$ alkenyl group containing monoglycidyl ether and one or more $C_{15}$ alkenyl group containing diglycidyl ether in the presence of a free radical inhibitor to at least partially fractionate the diglycidyl ether from the monoglycidyl ether.

2. The method as claimed in claim 1, wherein the free radical inhibitor is chosen from a phenol having one bulky group *ortho* to the OH group, a phenol having two bulky groups *ortho* to the OH group, and an aryl benzofuranone.

3. The method as claimed in claim 1, wherein the mixture of one or more $C_{15}$ alkenyl group containing monoglycidyl ether and one or more $C_{15}$ alkenyl group containing diglycidyl ether has up to 30 wt.%, based on the total solids of the epoxy resin mixture, of a $C_{15}$ alkenyl group containing monoglycidyl ether.

4. The method as claimed in claim 1, further comprising forming the mixture of one or more $C_{15}$ alkenyl group containing monoglycidyl ether and one or more $C_{15}$ alkenyl group containing diglycidyl ether by epoxidizing a mixture of one or more cardol and one or more cardanol with an epihalohydrin prior to the distilling.

5. The method as claimed in any one of claims 1, 2, 3 or 4, wherein the resulting epoxy resin composition comprises 15 wt.% or less of oligomers resulting from side chain coupling reactions of alkenyl groups, based on the total solids of the given epoxy resin composition.

6. The method as claimed in any one of claims 1, 2, 3 or 4, wherein the resulting epoxy resin composition comprises 10 wt.% or less of oligomers resulting from side chain coupling reactions of alkenyl groups, based on the total solids of the given epoxy resin composition.

7. The method as claimed in claim 1, further comprising reacting the one or more $C_{15}$ alkenyl group containing diglycidyl ether with a polyamine to form a polyamine adduct of the $C_{15}$ alkenyl group containing diglycidyl ether.

8. The method as claimed in claim 7, wherein the polyamine is diethylenetriamine.

9. A low viscosity and low VOC epoxy resin composition comprising an adduct of a polyamine and one or more $C_{15}$

alkenyl group containing diglycidyl ether.

10. A rapid cure liquid coating composition comprising an adduct of a polyamine and one or more $C_{15}$ alkenyl group containing diglycidyl ether and having a solids content of 85 wt. % solids or higher, based on the total weight of the composition.

Europäisches
Patentamt

European
Patent Office

Office européen
des brevets

## EUROPEAN SEARCH REPORT

Application Number

EP 14 19 2129

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/138994 A1 (DOW GLOBAL TECHNOLOGIES LLC [US]; WU YAN [CN]; CHEN CHEN [CN]; SHEN YU) 26 September 2013 (2013-09-26) | 9,10 | INV. C08G59/22 C08J3/00 C08G59/24 C09D163/00 C08L63/00 |
| A | * claims 9,10 * ----- | 1-8 | |

TECHNICAL FIELDS
SEARCHED      (IPC)

C08G
C08J
C09D
C08L

The present search report has been drawn up for all claims

| Place of search | Date of completion of the search | Examiner |
|---|---|---|
| The Hague | 10 April 2015 | Siemens, Thomas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

**ANNEX TO THE EUROPEAN SEARCH REPORT**
**ON EUROPEAN PATENT APPLICATION NO.**                    EP 14 19 2129

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2015

| Patent document cited in search report | Publication date | Patent family member(s) | Publication date |
|---|---|---|---|
| WO 2013138994 A1 | 26-09-2013 | CN 104204085 A<br>EP 2812394 A1<br>KR 20140143408 A<br>US 2015051317 A1<br>WO 2013138994 A1 | 10-12-2014<br>17-12-2014<br>16-12-2014<br>19-02-2015<br>26-09-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10218973 A
- US 2985622 A, Leppert
- US 5208317 A
- US 5109099 A
- US 4981926 A

**Non-patent literature cited in the description**

- **JAY, R.R.** Direct Titration of Epoxy Compounds and Aziridines. *Analytical Chemistry,* March 1964, vol. 36 (3), 667-668